# EUROPÄISCHE PATENTSCHRIFT

(11) **EP 1 819 652 B1**
(45) Veröffentlichungstag und Bekanntmachung des Hinweises auf die Patenterteilung: **13.08.2008**
(21) Anmeldenummer: 05850234.5
(22) Anmeldetag: 07.12.2005
(51) Int. Cl.: C07C 29/141, C07C 31/26

(54) **VERFAHREN ZUR HERSTELLUNG VON ZUCKERALKOHOLEN**
METHOD FOR THE PRODUCTION OF SUGAR ALCOHOLS
PROCEDE DE FABRICATION D'ALDITOLS

(30) Priorität: 07.12.2004 DE 102004058811
(43) Veröffentlichungstag der Anmeldung: 22.08.2007
(73) Patentinhaber: Fraunhofer-Gesellschaft zur Förderung der angewandten Forschung e.V., 80686 München (DE)
(72) Erfinder: HIRTH, Thomas, 77815 Bühl (DE); SCHWEPPE, Rainer, 76228 Karlsruhe (DE); GRAF, Jürgen, 76327 Pfinztal (DE); BUSCH, Rainer, 76530 Baden-Baden (DE); PURSCH, Matthias, 77836 Rheinmünster (DE)
(86) Internationale Anmeldenummer: PCT/EP2005/013085
(87) Internationale Veröffentlichungsnummer: WO 2006/061196

(56) Entgegenhaltungen:
- WO-A-20/05021475
- DE-A1- 10 258 089
- GB-A- 806 236
- US-A- 4 380 680
- US-A- 4 413 152
- US-A- 4 471 144
- US-A- 4 487 980
- US-A- 4 520 211
- US-A1- 2002 133 048
- US-B1- 6 235 797
- ARENA: "Deactivation of ruthenium catalysts in continuous glucose hydrogenation" APPL. CATAL. A, Bd. 87, 1992, Seiten 219-229, XP008060949
- GALLEZOT P ET AL: "Glucose Hydrogenation on Ruthenium Catalysts in a Trickle-Bed Reactor" JOURNAL OF CATALYSIS, ACADEMIC PRESS, DULUTH, MN, US, Bd. 180, Nr. 1, 15. November 1998 (1998-11-15), Seiten 51-55, XP004449461 ISSN: 0021-9517
- KUSSEROW ET AL: "Hydrogenation of Glucose to Sorbitol over Nickel and Ruthenium Catalysts" ADV. SYNTH. CATAL., Bd. 345, Nr. 1/2, 2003, Seiten 289-299, XP002370441

## Beschreibung

Die Erfindung betrifft ein Verfahren zur Herstellung von Polyalkoholen in Form von Zuckeralkoholen aus der Gruppe Sorbit und/oder Mannit und gegebenenfalls weiteren C₆- und/oder C4- und/oder C₃-Polyolen und/oder C₂-Polyolen.

Zuckeralkohole finden vielfältig.industriell Verwendung. So wird beispielsweise Sorbit insbesondere als Zuckeraustauschstoff mit süßendem Charakter, z.B. für diätetische Lebensmittel, in der kosmetischen und pharmazeutischen Industrie oder auch zur technischen Anwendung in der Papier- und Textilindustrie eingesetzt. Mannit wird beispielsweise ebenfalls als Zuckeraustauschstoff, als Füllstoff in der pharmazeutischen Industrie, bei der Herstellung von synthetischen Harzen, etc. eingesetzt. Ferner sind Polyole mit bis zu sechs Kohlenstoffatomen, wie Propandiole, Propantriol (Glycerin) und Butandiole, von großer technischer Relevanz und kommen beispielsweise in der Petrochemie als Grundstoff für die Herstellung von Kunststoffen zum Einsatz. Solche Polyole werden gegenwärtig aus fossilen Brennstoffen, insbesondere Erdöl, gewonnen und nach Fraktionierung der Kunststoffsynthese, wie zur Herstellung von Polyurethanen, zugeführt. Mit "Polyolen" sind in diesem Zusammenhang im übrigen organische Alkohole mit wenigstens zwei Hydroxygruppen angesprochen.

Zur Herstellung von Zuckeralkoholen sind biochemische Verfahren bekannt, bei welchen Zuckeralkohole durch enzymatische Behandlung der entsprechenden Monosaccharide erzeugt werden, wobei jedoch sehr hohe Reaktionszeiten von mehreren Stunden oder Tagen erforderlich sind, um eine hohe Ausbeute an Zuckeralkohol zu erhalten.

Die Herstellung von Zuckeralkoholen geschieht daher in der Regel durch katalytische Hydrierung von Sacchariden oder anderen Carbonylverbindungen mit Wasserstoff bei erhöhtem Druck und erhöhter Temperatur. Dabei sind neben diskontinuierlichen bzw. chargenweise durchgeführten Verfahren, bei welchen die Reaktionsmischung im allgemeinen über mehrere Stunden hinweg gerührt wird, kontinuierliche Verfahren bekannt, bei welchen die Reaktionsmischung in Rohrreaktoren mit dem Hydrierkatalysator in Kontakt gebracht wird.

Die DE 1 002 304 A beschreibt ein Verfahren zur kontinuierlichen Hydrierung von reduzierbaren Zuckern, indem ein feinpartikulärer Nickelkatalysator suspendiert und mit einer wäßrigen Eduktlösung gemischt und die Mischung bei erhöhtem Druck und erhöhter Temperatur kontinuierlich mit einem Wasserstoffstrom in Kontakt gebracht wird. Als Edukte werden insbesondere Glucose, invertierter Rohrzucker oder Laktose eingesetzt, welche auf die vorgenannte Weise zu Sorbit und Mannit (aus Glucose oder invertiertem Rohrzucker) oder Laktosit (aus Laktose) umgesetzt werden. Nachteilig sind einerseits die für einen zufriedenstellenden Umsatz erforderlichen hohen Reaktionszeiten, andererseits die verhältnismäßig geringe Selektivität zur Erzeugung der spezifischen Zuckeralkohole.

Der DE 960 352 B ist ein Verfahren zur Herstellung einer Mischung der Zuckeralkohole Sorbit und Mannit entnehmbar, indem eine wäßrige Saccharoselösung bei erhöhtem Druck und erhöhter Temperatur in Gegenwart eines Nickelkatalysators hydrolysiert wird. Nachteilig ist auch hier insbesondere die erhebliche Reaktionszeit von 45 min bis zu mehreren Stunden, um einen zufriedenstellenden Umsatz der Hydrolyse-reaktion zu erreichen, wobei letztere abgebrochen wird, wenn sie in einem Bereich zwischen 95% und 99% vollständig ist. Ferner ist die Selektivität bezüglich bestimmter Vertreter von Zuckeralkoholen relativ geringe.

Die DE 199 29 368 A1 beschreibt ein Verfahren zur Herstellung des Zuckeralkohols Mannit aus Fructose, wobei die Fructose kontinuierlich in Gegenwart eines Raney-Nickel-Katalysators bei einer Temperatur zwischen 50°C und 180°C und bei einem Druck zwischen 50 bar und 300 bar hydriert: wird. Die Fructose wird im wäßriger Lösung eingesetzt; als Hydriermittel kommt Wasserstoff zum Einsatz. Während zu der für eine zufriedenstellende Umsetzung des Eduktes erforderlichen Reaktionszeit keine Angaben gemacht werden, ist auch hier die Selektivität recht gering und lassen sich insbesondere ausschließlich Gemische aus Mannit und Sorbit herstellern.

In der DE 100 65 029 A1 geht es um ein ähnliches Verfahren zur Herstellung von Alkoholen, insbesondere von Zuckeralkoholen, durch Umsetzung von Carbonylverbindungen, insbesondere Zuckern, wie Sorbit aus Dextrose, Sorbit und Mannit aus Fructose, Xylit aus Xylose, Maltit aus Maltose, Isomaltit aus Isomaltose, Dulcit aus Galaktose und Lactit aus Lactose. Dabei werden die Edukte in Gegenwart eines Raney-katalysator auf der Basis von Nickel, Kobalt, Kupfer, Eisen, Platin, Palladium oder Ruthenium in wäßriger Lösung kontinuierlich mit Wasserstoff zur Umsetzung gebracht. Es wird ein Druck zwischen 30 bar und 450 bar sowie eine Temperatur von höchstens 150°C eingestellt, um im Falle des Einsatzes von Zuckern als Edukte diese nicht zu karamelisieren. Was die Herstellung von Zuckeralkoholen betrifft, so werden weder Angaben zur Reaktionszeit noch zur Selektivität gemacht, wobei aufgrund der erwähnten Gefahr einer Karamelisierung bei Temperaturen-oberhalb 150°C und einer mehrstufigen Hydrierung von verhältnismäßig langen Reaktionszeiten ausgegangen werden muß.

Die DE 1 931 112 A1 beschreibt ein Verfahren zur Herstellung von Mannit und Sorbit aus Saccharose, indem eine Saccharoselösung bei einer Temperatur von etwa 160°C bis 190°C und bei einem Druck von etwa 35 bis 211 bar mit Wasserstoff in Gegenwart eines Hydrierkatalysators hydriert wird, wobei das Verfahren kontinuierlich durchgeführt werden kann. Als Hydrierkatalysatoren sind Metallkatalysatoren auf der Basis von Nickel erwähnt. Die Verweilzeit soll 15 min bis 2,5 Stunden betragen.

Der EP 0 773 063 A1 ist ein weiteres Verfahren zur Hydrierung von reinen Zuckeralkoholen unter Verwendung eines Hydrierkatalysators in Form von Raney-Nickel entnehmbar. Als Verfahrensparameter sind z.B. Temperaturen von 110°C bis 150°C und Drucke von 40 bar bis 200 bar erwähnt. Gemäß einem Ausführungsbeispiel wird kristalline Glucose bei 130°C und 150 bar im Durchstrom durch einen Reaktor kontinuierlich an Raney-Nickel hydriert, wobei eine Ausbeute an Sorbit von 99,3% erhalten wird. Die Verwendung eines Katalysetors auf der Basis von Ruthetium/Ruthetiumoxid ist nicht vorgesehen, da sie bei einer Verfahrensführung gemäß der EP 0 773 063 A1 angeblich zu einer-Isomerisierung, Zersetzung und Polymerisierung bei der Hydrolase führt.

Die US 4 520 211 A beschreibt ein kontinuierliches Verfahren zur Herstellung von Polyalkoholen durch Hydrierung von in wäßriger Lösung befindlichen Kohlenhydraten an einem Festbettkatalysator auf der Basis von Ruthenium bei erhöhten Temperaturen und erhöhten Drucken. Als Träger für den Ru-Katalysator ist unter anderem Aluminiumoxid erwähnt. Als vorteilhafte Temperaturen werden solche im Bereich von 60°C bis 200°C angegeben, während als vorteilhafte Drucke solche im Bereich von 25 bar bis 300 bar genannt sind. Die eingesetzten Kohlenhydrate werden aus der Gruppe der Mono- und Polysaccharide gewählt. Unter anderem wird eine solchermaßen durchgeführte Hydrierung von Glucose bei 150°.C und 250 bar unter Erhalt eines Umsatzes von 99% zu Sorbit bei einer Ausbeute von 97% beschrieben. Nachteilig ist indes insbesondere wiederum die erhebliche Reaktionszeit, die sich für die vorgenannte Hydrierung von Glucose zu Sorbit zu einem Wert von mehr als zwei Stunden errechnet.

Die US 6 235 7'97 B1 beschreibt ebenfalls ein Verfahren zur kontinuierlichen Hydrierung einer wäßrigen Glucoselösung an einem geträgerten Rutheniumkatalysator zu Sorbit bei einem Umsatz von wenigstens 98% und einer Selektivität (für Sorbit) von wenigstens 95%. Den in der Patentschrift im Rahmen von Ausführungsbeispielen angegebenen Reaktionsbedingungen sind Temperaturen zwischen 100°C und 120°C sowie Drucke zwischen etwa 130 bar und etwa 160 bar zu entnehmen. Für die Reaktionszeiten ergeben sich indes wiederum Werte von jedenfalls mehr als einer halben Stunde.

Entsprechendes gilt weitgehend für die GB 806 236 A, welche bei einem ähnlichen Verfahren Reaktionszeiten von wenigstens 2,5 h bis 24 h angibt, wobei ein Druck von höchstens etwa 100 bar eingestellt wird.

Der Erfindung liegt die Aufgabe zugrunde, ein Verfahren zur Herstellung von Polyalkoholen der eingangs genannten Art dahingehend weiterzubilden, daß es bei einer hohen Selektivität für die gewünschten Zuckeralkohole, insbesondere Sorbit und/oder Mannit, sowie gegebenenfalls für die weiteren C₂- bis C₆-Polyole kostengünstig und effektiv ist und insbesondere eine einfache Steuerung der Selektivität hinsichtlich der jeweils gewünschten Produkte ermöglicht.

Erfindungsgemäß wird diese Aufgabe bei einem Verfahren der eingangs genannten Art dadurch gelöst, daß wenigstens ein wenigstens eine Glucose- und/oder wenigstens eine Fructose-einheit enthaltendes Mono-, Di-, Oligo- und/oder Polysaccharid in Gegenwart eines hydrierenden Katalysators auf der Basis von Ruthenium (Ru) und/oder Rutheniumoxid in wäßriger Phase bei erhöhter Temperatur und erhöhtem Druck mit Wasserstoff unter Erhalt der genannten Polyalkohole kontinuierlich zur Umsetzung gebracht wird, wobei eine Temperatur zwischen 120°C und 280°C, ein Druck von wenigstens 150 bar und eine Verweilzeit der Reaktanden bei der katalytische Hydrierung von höchstens 400 s eingestellt wird.

Überraschenderweise wurde gefunden, daß sich bei einer solchen, sehr kurzen Verweilzeit, d.h. bei einer sehr kurzen Kontaktzeit der genannten Edukte in wäßriger Phase mit dem Wasserstoff in Gegenwart des hydrierenden Katalysators auf der Basis von Ruthenium/Rutheniumoxid, von höchstens 400 s, ein praktisch vollständiger Umsatz der Edukte erzielen läßt, ohne daß diese auch bei einer Temperatur im Bereich von oder oberhalb etwa 150°C karamelisiert oder anderweitig beeinträchtigt werden. Die Verweilzeit beträgt dabei besonders bevorzugt zwischen etwa 5 s und etwa 360 s, beispielsweise im Bereich von etwa 180 bis etwa 240 s.

Dabei wird erfindungsgemäß je nach gewünschtem Produktspektrum als Edukt wenigstens ein wenigstens eine Glucose- und/oder Fructoseeinheit enthaltendes Mono-, Di-, Oligo- und/oder Polysaccharid; z.B. vorzugsweise Glucose und/oder Fructose, eingesetzt. Ferner können beispielsweise auch zumindest eine Glucose- und/oder Fructoseeinheit enthaltende Di-, Oligo- und/oder Polysaccharide zum Einsatz kommen. Bei letzterer Alternative kann es sich als vorteilhaft erweisen, wenn ein sowohl wenigstens eine Glucose- als auch wenigstens eine Fructoseeinheit enthaltendes Di-, Oligo- und/oder Polysaccharid, insbesondere Saccharose (Glucose-Fructose) und/oder Raffinose (Glucose-Fructose-Galactose), eingesetzt wird.

Wird, wie weiter unten näher erläutert, ein solches Edukt (z.B. Glucose) eingesetzt, so läßt sich bei einer geeigneten Steuerung der Verfahrensparameter eine Selektivität des jeweiligen Produktes (z.B. Sorbit) von nahezu 100% einstellen, wobei insbesondere durch Variation der Temperatur, Druck, der kurzen Verweilzeit und/oder der Wasserstoffs- bzw. Eduktkonzentration die Selektivität für die anderen möglichen Produkte (z.B. Mannit bzw. die kurzkettigeren Polyalkohole) gezielt gesteuert werden kann.

Mit den erfindungsgemäß eingesetzten Katalysatoren auf der Basis von Ruthenium (Ru) und/oder Rutheniumoxid kann bei den genannten, kurzen Verweilzeiten ein praktisch vollständiger Umsatz eines Glucose und/oder Fructose enthaltenden Eduktes bei einer (je nach Temperatur bzw. Druck, kurzer Verweilzeit und/oder Wasserstoff- bzw. Eduktkonzentration) sehr hohen Selektivität von Sorbit und/oder Mannit und/oder demgegenüber kurzkettigeren Polyolen erzielt werden. Die , , genannten Katalysatoren auf der Basis von Ruthenium (Ru) und/oder Rutheniumoxid haben sich im Hinblick auf einen vollständigen Umsatz des Eduktes und auf eine äußerst hohe Selektivität bezüglich der vorgenannten Produkte überraschenderweise als anderen bekannten Hydrierkatalysatoren deutlich überlegen erwiesen. Dabei haben sich insbesondere Mischkatalysatoren als vorteilhaft erwiesen, welche sowohl Ruthenium als auch Rutheniumoxid enthalten und welche vorzugsweise an wenigstens einem Träger, wie Aluminiumoxid (Al₂O₃)_{'} immobilisiert sind. Ein weiterer Vorteil solcher Katalysatoren auf der Basis von Ruthenium bzw. Rutheniumoxid z.B. gegenüber einem zur Herstellung von Zuckerälkoholen verbreitet eingesetzten Katalysator auf der Basis von. Raney-Nickel - besteht darin, daß ihre Aktivität über einen sehr langen Zeitraum praktisch konstant bleibt und sie nicht toxisch und folglich einfach handhabbar sind.

Bezüglich des während der kontinuierlichen Hydrierungsreak- " tion eingestellten Druckes hat sich insbesondere ein Druck von wenigstens 200 bar, vorzugsweise von wenigstens 220 bar, als vorteilhaft erwiesen. Besonders bevorzugt sind Werte zwischen etwa 220 bar und etwa 280 bar, insbesondere zwischen etwa 230 bar und etwa 270 bar, beispielsweise im Bereich von etwa 250 bar.

Bei dem erfindungsgemäßen Verfahren wird, wie bereits erwähnt, eine Temperatur zwischen 120°C und 280°C eingestellt, wobei die Temperatur z.B. im Falle der Herstellung von Sorbit aus Glucose oder von Sorbit und Mannit aus Fructose vorzugsweise im Bereich zwischen etwa 120°C und etwa 180°C, insbesondere zwischen etwa 130°C und etwa 170°C, beispielsweise im Bereich von etwa 150°C eingestellt wird. Dabei läßt sich insbesondere im Falle eines Einsatzes eines eduktes in Form von Glucose eine Selektivität für Sorbit von nahezu 100% bei einem praktisch vollständigen Umsatz des Eduktes erzielen läßt. Ist hingegen der Erhalt eines höheren Anteils an Mannit aus Glucose von Interesse, so kann die Temperatur vorzugsweise bis auf etwa 280°C, insbesondere bis auf etwa 250°C, z.B. bis auf etwa 225°C erhöht werden, um die Selektivität für Mannit gegenüber der von Sorbit zu erhöhen. Werden Di-, Oligo- und/oder Polysaccharide eingesetzt, wie z.B. ein Disaccharid aus einer Glucose- und einer Fructoseeinheit (Saccharose) oder ein Trisaccharid aus einer Glucose-, einer Fructose- und einer Galactoseeinheit (Raffinose), so beträgt der bevorzugte Temperaturbereich etwa 175°C bis 225°C, vorzugsweise im Bereich von etwa 200°C, um das eingesetzte Edukt zunächst im wesentlichen vollständig zu spalten. Im Falle einer Erhöhung der Temperatur auf etwa 225°C oder mehr läßt sich die Selektivität des erfindungsgemäßen Verfahrens jeweils zu den C₂-, C₃-, C₄- und/oder C₆-Polyolen, wie insbesondere 1,3-Propandiol, Glycerin (1,2,3-Propantriol), 2,3-Butandiol, 1,4-Butandiol, 1,2-Ethandiol und gegebenenfalls weiteren partiell hydrierten Zuckeralkoholen in kleineren Mengen, hin verschieben.

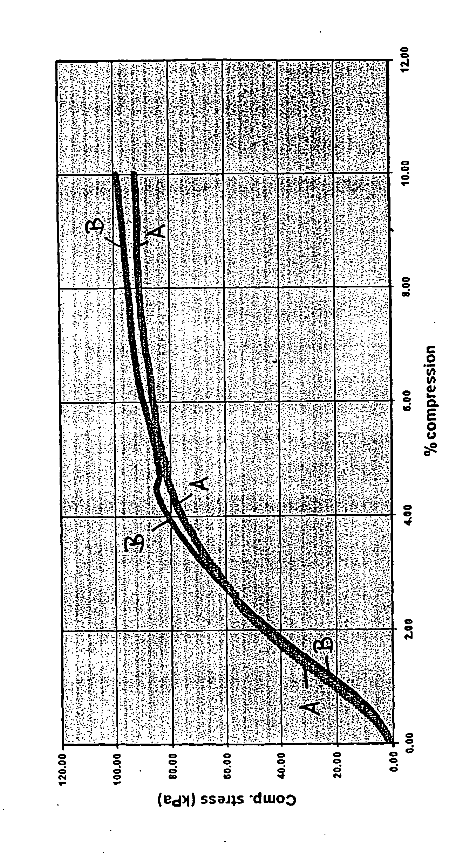

Wie bereits angedeutet, gibt das erfindungsgemäße Verfahren folglich die Möglichkeit, daß die Produktanteile der Zukkeralkohole Sorbit und Mannit bzw. der genannten Polyalkohole durch Variation wenigstens eines der Parameter Temperatur, insbesondere in einem Bereich zwischen 120°C und 280°C bzw. den vorgenannten, bevorzugten Temperaturbereichen, Druck, Verweilzeit und/oder Wasserstoff- bzw. Eduktkonzentration eingestellt werden. Die jeweilige Selektivität läßt sich auf diese Weise also bei einem praktisch vollständigen Umsatz der Edukte zwischen nahezu 100% für Sorbit über einen überwiegenden Anteil an Mannit bis hin zu den kurzkettigeren Polyalkoholen, wie z.B. 1,3-Propandiol, Propantriol (Glycerin), 2,3-Butandiol, 1,4-Butandiol und Ethandiol, steuern. Was die letztgenannten Polyalkohole betrifft, so wurde des weiteren überraschenderweise gefunden, daß sich mittels des erfindungsgemäßen Verfahrens Produktspektren solcher Alkohole herstellen lassen, welche bei der Herstellung von Polyurethanen unmittelbar aus der erhaltenen Mischung bessere Ergebnisse zu liefern vermögen als es bei der Herstellung von Polyurethanen aus der entsprechenden, aus Erdöl erhaltenen Produktpalette gemäß dem Stand der Technik der Fall ist. So zeigt das in der beigefügten Zeichnung dargestellte Schaubild, daß die Druckspannung ("Comp. stress" in [kPa]) in Polyurethanen (Kurve A), welche unmittelbar durch Polymerisation aus den mittels des erfindungsgemäßen Verfahrens erzeugten Polyolen hergestellt worden sind (Experiment E9 des Beispiels 3 weiter unten), in Abhängigkeit des Druckes ("compression" in [%]) geringer ist als in Polyurethanen (Kurve B), welche unmittelbar durch Polymerisation eines die entsprechenden Polyole enthaltenden, kommerziellen Erdölproduktes hergestellt worden sind.

Als Eduktkonzentration hat sich insbesondere im Falle einer Herstellung von Sorbit und/oder Mannit eine Konzentration an Edukten, z.B. Glucose, zwischen 5 und 50 Mass.-%, insbesondere zwischen 10 und 40 Mass.-%, bezogen auf den Gesamtstrom, also Edukt einschließlich des Lösungsmittels Wasser, als vorteilhaft erwiesen. Indes kann die Eduktkonzentration auch anders gewählt werden, z.B. insbesondere kleiner als 5 Mass.-%, sofern vornehmlich die anderen Polyalkohole erzeugt werden sollen.

Nachstehend ist das erfindungsgemäße Verfahren anhand von Ausführungsbeispielen näher erläutert. Bei allen Ausführungsbeispielen (Tabellen 2, 4, 6, 8, 10, 12 und 14) bedeuten
- TOC:: Gesamter Organischer Kohlenstoff (Total Organic Carbon);
- Ausbeute:: jeweils bei einem Umsatz an Edukt (hier Glucose bzw. Fructose bzw. Saccharose) von 100%, d.h. die Ausbeute entspricht der Selektivität; sämtliche Ausbeuten sind massenspezifisch unter Berücksichtigung des höheren Molekulargewichtes des - hydrierten - Produktes gegenüber dem Edukt angegeben.

### Beispiel 1

Kontinuierliche Herstellung der Zuckeralkohole Sorbit und/oder Mannit und gegebenenfalls weiterer Polyalkohole durch Hydrierung von Glucose in wäßriger Lösung mit gasförmigem Wasserstoff in Gegenwart eines Ruthenium/Rutheniumoxid-Mischkatalysators auf Aluminiumoxid in Form eines in den Reaktor eingebrachten Festbettes:

In einem Rohrreaktor aus Edelstahl mit einer Reaktorlänge von zwei mal 163 cm (parallel geschaltet) mit 9 mm Innendurchmesser wird das Monosaccharid Glucose als Edukt in wäßriger Lösung mit gasförmigem Wasserstoff kontinuierlich zur Umsetzung gebracht. In dem Rohrreaktor befindet sich ein Festbett eines Ruthenium/Rutheniumoxid-Mischkatalysators auf Partikeln von Aluminiumoxid als Träger. Das Experiment wird vier mal, d.h. jeweils zwei mal bei 150°C und bei 200°C, durchgeführt, wobei die in der nachfolgenden Tabelle 1 zusammengestellten Parameter eingestellt werden.

**Tabelle 1:**

| Experiment | E1 | E2 | E3 | E4 |
|---|---|---|---|---|
| Glucosekonzentration der eingesetzten Lösung [Mass.-% Glucose in wäßriger Lösung] | 30 | 30 | 30 | 30 |
| Glucosekonzentration im Reaktor [Mass.-% Glucose in Gesamtmassenstrom Reaktor] | 5 | 5 | 20 | 20 |
| eingesetzte Wasserstoffkonzentration [Mol H₂ pro Mol eingesetzter Glucose] | 100 | 100 | 25 | 25 |
| Verweilzeit im Reaktor [Sekunden, s] | 300 | 300 | 300 | 300 |
| Temperatur im Reaktor [°C] | 200 | 200 | 150 | 150 |
| Druck im Reaktor [bar] | 250 | 250 | 250 | 250 |
| Massenströme im Reaktor: | | | | |
| a) Gesamtmassenstrom [kg/h] | 0,946 | 0,946 | 0,998 | 0,998 |
| b) Massenstrom Wasser [ml/min] | 12,612 | 12,612 | 5,074 | 5,074 |
| c) Massenstrom Glucose (30%ig) [ml/min] | 2,368 | 2,368 | 9,996 | 9,996 |
| d) Massenstrom Wasserstoff [ml/min] | 29,166 | 29,166 | 30,786 | 30,786 |
| pH-Wert | 6 | 6 | 6 | 6 |

Zur Durchführung der Experimente E1 bis E4 wird der Reaktor mit dem in diesem in Form des Festbettes enthaltenen hydrierenden Katalysator zunächst angefahren, indem der Reaktor bei dem jeweiligen Reaktionsdruck von 250 bar und der jeweiligen Reaktionstemperatur von 150°C bzw. 200°C mit einem Strom reinen Wassers beaufschlagt wird. Ist ein konstanter Wasserstrom bei den genannten Druck- und Temperaturbedingungen erreicht worden, so wird der Reaktor ferner mit einem Wasserstoffstrom beaufschlagt, um den Hydrierkatalysator zu reduzieren und somit zu aktivieren. Hat sich ein konstanter Wasserstrom und ein konstanter Wasserstoffstrom mit dem jeweiligen, in Tabelle 1 wiedergegebenen Wert eingestellt, so wird die 30%ige Glucoselösung zugesetzt, wobei jeweils der in Tabelle 1 genannte Massenstrom an Glucose eingestellt wird. Dabei wird die Glucosekonzentration im Reaktor beim vorliegenden Ausführungsbeispiel mittels Dosierpumpen derart eingestellt, daß sich ein Wert von 5 (Experimente E1 und E2) bzw. 20 Mass.-% (Experimente E3 und E4) bezogen auf den dem Reaktor zugesetzten Gesamtmassenstrom ergibt. Die Verweilzeit von 300 s bzw. 5 min kann ebenfalls - unter Berücksichtigung der Dichte des Mediums bei den im Reaktor herrschenden Bedingungen und dem jeweiligen Volumenstrom - durch entsprechende Steuerung der zugeordneten Dosierpumpe eingestellt werden.

In der nachfolgenden Tabelle 2 sind die im Rahmen der Experimente erhaltenen Ergebnisse anhand des Umsatzes der als Edukt eingesetzten Glucose sowie anhand der Ausbeuten der als Produkte erhaltenen Zuckeralkohole Sorbit, Mannit und weiterer Polyalkohole zusammengestellt.

**Tabelle 2:**

| Experiment | E1 | E2 | E3 | E4 |
|---|---|---|---|---|
| Wiederfindungsrate an Kohlenstoff (TOCP_{Produkte} ¹⁾/TOC_{Edukte} ¹⁾ in [%]) | 89 | 83 | 96 | 97 |
| Umsatz an Glucose [%] | 100 | 100 | 100 | 100 |
| Ausbeute²) an Sorbit [%] | 45 | 62 | 98 | 98 |
| Ausbeute²) an Mannit [%] | 21 | 13 | < 1,5 | < 1,5 |
| Ausbeute an C₆-Alkoholen [%] | 13 | 8 | < 0,5 | < 0,5 |
| Ausbeute an C₃-/C₄-Alkoholen [%] | 13 | 8 | 0 | 0 |

Die Experimente E1 bis E4 zeigen, daß bei einer Verweilzeit von 300 s, einem Druck von 250 bar und einer Temperatur von 150°C ein vollständiger Glucoseumsatz in Höhe von 100% bei der kontinuierlichen katalytischen Hydrierung der Glucose mit Wasserstoff an Ruthenium/Rutheniumoxid auf Aluminiumoxid erzielt werden kann. Dabei ergibt sich eine äußerst hohe Ausbeute bzw. Selektivität des Zuckeralkohols Sorbit in Höhe von 98%, während sich für den Zuckeralkohol Mannit eine geringe Ausbeute bzw. Selektivität von maximal 1,5% ergibt. Als weitere Reaktionsprodukte können lediglich andere C₆-Alkohole in äußerst geringen Anteilen von maximal 0,5% nachgewiesen werden.

Wie weiterhin ersichtlich ist, läßt sich die Ausbeute an dem Zuckeralkohol Mannit sowie von weiteren Polyolen, insbesondere C₃- und C₄-Polyalkoholen, gegenüber Sorbit dadurch erhöhen, indem die Temperatur auf einen gegenüber etwa 150°C größeren Wert, hier 200°C, erhöht wird, wobei sich ein Produktverhältnis von Sorbit zu Mannit zwischen etwa 1:2 und etwa 1:3 einstellt, ohne daß die eingesetzte Glucose aufgrund der erfindungsgemäß sehr kurzen Verweilzeit im Reaktor karamelisiert wird. Ferner fallen im Falle einer solchen Temperaturerhöhung auf 200°C weitere C₃-, C₄- und C₆-Alkohole mit einem Gesamtanteil von etwa 20% an. Der Anteil an C₃- und C₄-Polyalkoholen beträgt in der Größenordnung von 10% bezogen auf die gesamte Produktmenge und setzt sich praktisch ausschließlich aus dem C₃-Polyalkohol 1,3-Propandiol sowie aus den _{C4}-Polyalkoholen 2,3-Butandiol und 1,4-Butandiol zusammen. Auch bei dieser Temperatur wird ein vollständiger Umsatz der eingesetzten Glucose in Höhe von 100% festgestellt.

Wie aus dem nachfolgenden Beispiel 2 ersichtlich ist, nimmt die Ausbeute an kurzkettigen Alkoholen grundsätzlich bei zunehmender Temperatur infolge thermischer Spaltung zu, wobei im Falle der Herstellung von Sorbit aus Glucose ein Druck von etwa 250 bar, eine Temperatur von etwa 150°C und eine Verweilzeit von etwa 300 s optimale Verfahrensparameter darstellen. Eine vorteilhafte Konzentration an Wasserstoff liegt im Bereich zwischen etwa 10 und 200 Mol Wasserstoff pro Mol eingesetzter Glucose, vorzugsweise zwischen etwa 20 und 150 Mol Wasserstoff pro Mol eingesetzter Glucose.

### Beispiel 2

Kontinuierliche Herstellung der Zuckeralkohole Sorbit und Mannit sowie weiterer Polyalkohole durch Hydrierung von Glucose in wäßriger Lösung mit gasförmigem Wasserstoff in Gegenwart eines Ruthenium/Rutheniumoxid-Mischkatalysators auf Aluminiumoxid in Form eines in den Reaktor eingebrachten Festbettes:

In dem Rohrreaktor aus Edelstahl mit einer Reaktorlänge von zwei mal 163 cm (parallel geschaltet) mit 9 mm Innendurchmesser gemäß Beispiel 1 wird das Monosaccharid Glucose als Edukt in wäßriger Lösung mit gasförmigem Wasserstoff kontinuierlich zur Umsetzung gebracht. In dem Rohrreaktor befindet sich ein Festbett eines Ruthenium/Rutheniumoxid-Mischkatalysators auf Partikeln von Aluminiumoxid als Träger.

Das Experiment wird bei gegenüber Beispiel 1 höheren Temperaturen, nämlich je einmal bei 225°C und bei 250°C, durchgeführt, wobei die in der nachfolgenden Tabelle 3 zusammengestellten Parameter eingestellt werden.

**Tabelle 3:**

| Experiment | E5 | E6 |
|---|---|---|
| Glucosekonzentration der eingesetzten Lösung [Mass.-% Glucose in wäßriger Lösung] | 30 | 30 |
| Glucosekonzentration im Reaktor [Mass.-% Glucose in Gesamtmassenstrom Reaktor] | 5 | 5 |
| eingesetzte Wasserstoffkonzentration [Mol H₂ pro Mol eingesetzter Glucose] | 100 | 100 |
| Verweilzeit im Reaktor [Sekunden, s] | 300 | 300 |
| Temperatur im Reaktor [°C] | 225 | 250 |
| Druck im Reaktor [bar] | 250 | 250 |
| Massenströme im Reaktor: | | |
| a) Gesamtmassenstrom [kg/h] | 0,912 | 0,880 |
| b) Massenstrom Wasser [ml/min] | 12,180 | 11,748 |
| c) Massenstrom Glucose (30%ig) [ml/min] | 2,286 | 2,206 |
| d) Massenstrom Wasserstoff [ml/min] | 28,166 | 27,168 |
| pH-Wert | 6 | 6 |

Entsprechend Beispiel 1 wird der Reaktor zur Durchführung der Experimente E5 und E6 mit dem in diesem in Form des Festbettes enthaltenen hydrierenden Katalysator zunächst angefahren, indem der Reaktor bei dem jeweiligen Reaktionsdruck von 250 bar und der jeweiligen Reaktionstemperatur von 225°C bzw. 250°C mit einem Strom reinen Wassers beaufschlagt wird. Ist ein konstanter Wasserstrom bei den genannten Druck- und Temperaturbedingungen erreicht worden, wird der Reaktor ferner mit einem Wasserstoffstrom beaufschlagt, um den Hydrierkatalysator zu reduzieren und somit zu aktivieren. Hat sich ein konstanter Wasserstrom und ein konstanter Wasserstoffstrom mit dem jeweiligen, in Tabelle 3 wiedergegebenen Wert eingestellt, wird die 30%ige Glucoselösung zugesetzt, wobei jeweils der in Tabelle 3 genannte Massenstrom an Glucose eingestellt wird. Dabei wird die Glucosekonzentration im Reaktor beim vorliegenden Ausführungsbeispiel mittels Dosierpumpen derart eingestellt, daß sich ein Wert von 5 Mass.-% bezogen auf den dem Reaktor zugesetzten Gesamtmassenstrom ergibt. Die Verweilzeit von 300 s bzw. 5 min kann ebenfalls - unter Berücksichtigung der Dichte des Mediums bei den im Reaktor herrschenden Bedingungen und dem jeweiligen Volumenstrom - durch entsprechende Steuerung der zugeordneten Dosierpumpe eingestellt werden.

In der nachfolgenden Tabelle 4 sind die im Rahmen der Experimente erhaltenen Ergebnisse anhand des Umsatzes der als Edukt eingesetzten Glucose sowie anhand der Ausbeuten der als Produkte erhaltenen Zuckeralkohole Sorbit, Mannit und weiterer Polyalkohole zusammengestellt.

**Tabelle 4:**

| Experiment | E5 | E6 |
|---|---|---|
| Wiederfindungsrate an Kohlenstoff (TOC_{Produkte}¹⁾/TOC_{Edukte}¹⁾ in [%]) | 76 | 59 |
| Umsatz an Glucose [%] | 100 | 100 |
| Ausbeute²⁾ an Sorbit [%] | 35 | 11 |
| Ausbeute²⁾ an Mannit [%] | 18 | 12 |
| Ausbeute an C₆₋Alkoholen [%] | <9 | <3 |
| Ausbeute an C₃-/C₄-Alkoholen [%] | < 25 | < 34 |

Wie aus Tabelle 4 ersichtlich, läßt sich die Ausbeute an weiteren Polyolen, insbesondere C₃- und C₄-Polyalkoholen, gegenüber Sorbit dadurch erhöhen, indem die Temperatur auf einen Wert von etwa 225°C bzw. 250°C erhöht wird. Während sich bei 225°C (E5) ein Produktverhältnis von Sorbit zu Mannit von etwa 1:2 einstellt, ohne daß die eingesetzte Glucose aufgrund der erfindungsgemäß sehr kurzen Verweilzeit im Reaktor karamelisiert wird, erhöht sich der Anteil an C₃- und C₄-Polyalkoholen, welche sich praktisch ausschließlich aus den C₃-Polyalkoholen 1,3-Propandiol und Propantriol (Glycerin) sowie aus den C₄-Polyalkoholen 2,3-Butandiol und 1,4-Butandiol zusammensetzen, auf etwa 25% bezogen auf die gesamte Produktmenge. Der Anteil sonstiger, von Sorbit und Mannit verschiedener Polyalkohole beträgt kleiner 10%.

Im Falle einer Reaktionstemperatur von 250°C (E6) beträgt das Produktverhältnis von Sorbit zu Mannit etwa 1:1, wobei insbesondere der Produktanteil an Sorbit bei zunehmender Temperatur sinkt. Es ergibt sich eine erhöhte Ausbeute für die C₃- und C₄-Polyalkohole.in der Größenordnung von etwa einem Drittel der gesamten Produktmenge, wobei wiederum praktisch ausschließlich 1,3-Propandiol und Propantriol (Glycerin) sowie 2,3-Butandiol und 1,4-Butandiol erhalten werden. Der Anteil sonstiger, von Sorbit und Mannit verschiedener C₆-Alkohole sinkt auf etwa 3% ab.

Auch bei Temperaturen von 225°C und 250°C wird ein vollständiger Umsatz der eingesetzten Glucose in Höhe von 100% festgestellt. Eine vorteilhafte Konzentration an Wasserstoff liegt wiederum im Bereich zwischen etwa 10 und 200 Mol Wasserstoff pro Mol eingesetzter Glucose, vorzugsweise zwischen etwa 20 und 150 Mol Wasserstoff pro Mol eingesetzter Glucose.

### Beispiel 3

Kontinuierliche Herstellung der Zuckeralkohole Sorbit und Mannit sowie weiterer Polyalkohole durch Hydrierung von Glucose in wäßriger Lösung mit gasförmigem Wasserstoff in Gegenwart eines Ruthenium/Rutheniumoxid-Mischkatalysators auf Aluminiumoxid in Form eines in den Reaktor eingebrachten Fettbettes:

In dem Rohrreaktor aus Edelstahl mit einer Reaktorlänge von zwei mal 163 cm (parallel geschaltet) mit 9 mm Innendurchmesser gemäß den Beispielen 1 und 2 wird das Monosaccharid Glucose als Edukt in wäßriger Lösung mit gasförmigem Wasserstoff kontinuierlich zur Umsetzung gebracht. In dem Rohrreaktor befindet sich ein Festbett eines Ruthenium/Rutheniumoxid-Mischkatalysators auf Partikeln von Aluminiumoxid als Träger. Das Experiment wird jeweils bei einer Temperatur von 200°C und einer Verweilzeit von 300s, aber mit verschiedenen Konzentrationen an Wasserstoff bzw. Glucose durchgeführt, wobei die in der nachfolgenden Tabelle 5 zusammengestellten Parameter eingestellt werden.

**Tabelle 5:**

| Experiment | E7 | E8 | E9 | E10 |
|---|---|---|---|---|
| Glucosekonzentration im Reaktor [Mass.-% Glucose in Gesamtmassenstrom Reaktor] | 5 | 2 | 1 | 2 |
| eingesetzte Wasserstoffkonzentration [Mol H₂ pro Mol eingesetzter Glucose] | 100 | 500 | 1000 | 1000 |
| Verweilzeit im Reaktor [Sekunden, s] | 300 | 300 | 300 | 300 |
| Temperatur im Reaktor [°C] | 200 | 200 | 200 | 200 |
| Druck im Reaktor [bar] | 250 | 250 | 250 | 250 |

Entsprechend Beispiel 1 und 2 wird der Reaktor zur Durchführung der Experimente E7 bis E10 mit dem in diesem in Form des Festbettes enthaltenen hydrierenden Katalysator zunächst angefahren, indem der Reaktor bei dem jeweiligen Reaktionsdruck von 250 bar und der jeweiligen Reaktionstemperatur von 200°C mit einem Strom reinen Wassers beaufschlagt wird. Ist ein konstanter Wasserstrom bei den genannten Druck- und Temperaturbedingungen erreicht worden, wird der Reaktor ferner mit einem Wasserstoffstrom beaufschlagt, um den Hydrierkatalysator zu reduzieren und somit zu aktivieren. Hat sich ein konstanter Wasserstrom und ein konstanter Wasserstoffstrom mit dem jeweiligen, in Tabelle 5 wiedergegebenen Wert eingestellt, wird die Glucoselösung zugesetzt, wobei jeweils der in Tabelle 5 genannte Massenstrom an Glucose eingestellt wird. Dabei wird die Glucosekonzentration im Reaktor beim vorliegenden Ausführungsbeispiel mittels Dosierpumpen derart eingestellt, daß sich ein Wert zwischen 1 und 5 Mass.-% bezogen auf den dem Reaktor zugesetzten Gesamtmassenstrom ergibt. Die Verweilzeit von 300 s bzw. 5 min kann ebenfalls - unter Berücksichtigung der Dichte des Mediums bei den im Reaktor herrschenden Bedingungen und dem jeweiligen Volumenstrom - durch entsprechende Steuerung der zugeordneten Dosierpumpe eingestellt werden.

In der nachfolgenden Tabelle 6 sind die im Rahmen der Experimente erhaltenen Ergebnisse anhand des Umsatzes der als Edukt eingesetzten Glucose sowie anhand der Ausbeuten der als Produkt erhaltenen Zuckeralkohole Sorbit, Mannit und weiteren Polyalkohole zusammengestellt.

**Tabelle 6:**

| Experiment | E7 | E8 | E9 | E10 |
|---|---|---|---|---|
| Wiederfindungsrate an Kohlenstoff (TOC_{Produkte}¹⁾TOC_{Edukte}¹⁾ in [%]) | 93 | 68 | 99 | 80 |
| Umsatz an Glucose [%] | 100 | 100 | 100 | 100 |
| Ausbeute²⁾ an Sorbit [%] | 11,1 | 17,4 | 16,9 | 9,4 |
| Ausbeute²⁾ an Mannit [%] | 12,6 | 8,5 | 6,9 | 6,8 |
| Ausbeute an C₆-Alkoholen [%] | 6,4 | --- | --- | --- |
| Ausbeute an C₃-/C₄-Alkoholen [%] | 27 | 36 | 49 | 33 |

Exemplarisch wurden die im Experiment E9 mit einer Ausbeute von 49% erhaltenen C₃-/C₄-Alkohole auf die jeweiligen Vertreter derselben untersucht, wobei sich die folgende Zusammensetzung ergab (jeweils ausgedrückt als Ausbeute²⁾ des jeweiligen Polyalkohols) :
- Propantriol (Glycerin): 9,2%,
- 1,3-Propandiol: 10,6%,
- 2,3-Butandiol: 19,6% und
- 1,4-Butandiol: 9,4%.

Wie aus Tabelle 6 ersichtlich, lassen sich je nach Konzentration an eingesetzter Glucose bzw. an Wasserstoff in dem Reaktor bei den genannten Verfahrensparametern Ausbeuten an Sorbit und Mannit von insgesamt bis zu etwa 24% bezogen auf die gesamte Produktmenge und Ausbeuten an Polyolen aus der Gruppe Propantriol (Glycerin), 1,3-Propandiol, 2,3-Butandiol, 1,4-Butandiol und - im Falle des Experimentes E7 - weitere C₆-Polyole (nicht näher bestimmt) von insgesamt bis zu etwa 50% bezogen auf die gesamte Produktmenge erzielen, indem eine gegenüber Beispiel 2 höhere Wasserstoffkonzentration - hier zwischen 100 und 1000 Mol H₂ pro Mol eingesetzter Glucose eingestellt wird. Wiederum wird in allen Experimenten ein vollständiger Umsatz der eingesetzten Glucose erzielt.

Das Produktspektrum der Polyalkohole gemäß dem Experiment E9 wurde exemplarisch zur Herstellung von Polyurethan-Testkörpern durch Polymerisation verwendet, wobei die weiter oben erwähnten und in der beigefügten Zeichnung wiedergegebenen Ergebnisse (Kurve A) erhalten worden sind.

### Beispiel 4

Kontinuierliche Herstellung der Zuckeralkohole Sorbit und Mannit sowie weiterer Polyalkohole durch Hydrierung von Glucose in wäßriger Lösung mit gasförmigem Wasserstoff in Gegenwart eines Ruthenium/Rutheniumoxid-Mischkatalysators auf Aluminiumoxid in Form eines in den Reaktor eingebrachten Fettbettes:

In dem Rohrreaktor aus Edelstahl mit einer Reaktorlänge von zwei mal 163 cm (parallel geschaltet) mit 9 mm Innendurchmesser gemäß den Beispielen 1 bis 3 wird das Monosaccharid Glucose als Edukt in wäßriger Lösung mit gasförmigem Wasserstoff kontinuierlich zur Umsetzung gebracht. In dem Rohrreaktor befindet sich ein Festbett eines Ruthenium/Rutheniumoxid-Mischkatalysators auf Partikeln von Aluminiumoxid als Träger. Das Experiment wird bei einer konstanten Temperatur von 220°C und bei einer konstanten Wasserstoff-, bzw. Glucosekonzentration, aber mit verschiedenen Verweilzeiten von 150 s (Experiment E11) bzw. 300 s (Experiment E12) durchgeführt, wobei die in der nachfolgenden Tabelle 7 zusammengestellten Parameter eingestellt werden.

**Tabelle 7:**

| Experiment | E11 | E12 |
|---|---|---|
| Glucosekonzentration im Reaktor [Mass.-% Glucose in Gesamtmassenstrom Reaktor] | 5 | 5 |
| eingesetzte Wasserstoffkonzentration [Mol H₂ pro Mol eingesetzter Glucose] | 100 | 100 |
| Verweilzeit im Reaktor [Sekunden, s] | 150 | 300 |
| Temperatur im Reaktor [°C] | 220 | 220 |
| Druck im Reaktor [bar] | 250 | 250 |

Die Experimente E11 und E12 werden entsprechend Beispiel 1 bis 3 unter Berücksichtigung der vorgenannten Verfahrensparameter durchgeführt.

In der nachfolgenden Tabelle 8 sind die im Rahmen der Experimente E11 und E12 erhaltenen Ergebnisse anhand des Umsatzes der als Edukt eingesetzten Glucose sowie anhand der Ausbeuten der als Produkte erhaltenen Zuckeralkohole Sorbit, Mannit und weiterer Polyalkohole zusammengestellt.

**Tabelle 8:**

| Experiment | E11 | E12 |
|---|---|---|
| Wiederfindungsrate an Kohlenstoff (TOC_{Produkte}¹⁾/TOC_{Edukte}¹⁾ in [%]) | 82 | 65 |
| Umsatz an Glucose [%] | 100 | 100 |
| Ausbeute²⁾ an Sorbit [%] | 12,4 | 3,0 |
| Ausbeute²⁾ an Mannit [%] | 13,2 | 7,0 |
| Ausbeute an C₆-Alkoholen [%] | 6,3 | 2,8 |
| Ausbeute an C₃₋/C₄ Alkoholen [%] | 26 | 10 |

Wie der Tabelle 8 zu entnehmen ist, lassen sich bei einer Temperatur in dem Reaktor von 220°C die Ausbeuten an den weiteren Polyolen, insbesondere an den vorliegend gemessenen C₃-/C₄-Polyolen Propantriol (Glycerin), 1,3-Propandiol, 2,3-Butandiol und 1,4-Butandiol sowie an den nicht näher bestimmten C₆-Polyolen, durch Verlängerung der Verweilzeit bei ansonsten konstanten Verfahrensparametern nicht erhöhen, sondern ergibt eine Verdopplung der Verweilzeit von 150 s auf 300 s bei den genannten Verfahrensparametern eine Verringerung der Ausbeuten dieser Polyole um etwas mehr als die Hälfte. Zugleich nimmt die Ausbeute der Zuckeralkohole Sorbit und Mannit bei einem im wesentlichen konstanten Verhältnis von ca. 2:1 bis 2,5:1 um etwa die Hälfte ab. In beiden Fällen wird dabei ein vollständiger Umsatz der eingesetzten Glucose erreicht.

### Beispiel 5:

Kontinuierliche Herstellung der Zuckeralkohole Sorbit und Mannit und weiterer Polyalkohole durch Hydrierung von Glucose in wäßriger Lösung mit gasförmigem Wasserstoff in Gegenwart eines Ruthenium/Rutheniumoxid-Mischkatalysators auf Aluminiumoxid in Form eines in den Reaktor eingebrachten Fettbettes:

In dem Rohrreaktor aus Edelstahl mit einer Reaktorlänge von zwei mal 163 cm (parallel geschaltet) mit 9 mm Innendurchmesser gemäß den Beispielen 1 bis 4 wird das Monosaccharid Glucose als Edukt in wäßriger Lösung mit gasförmigem Wasserstoff kontinuierlich zur Umsetzung gebracht. In dem Rohrreaktor befindet sich ein Festbett eines Ruthenium/Rutheniumoxid-Mischkatalysators auf Partikeln von Aluminiumoxid als Träger. Das Experiment wird vier mal mit einer Verweilzeit von 5 s und jeweils bei 250°C, 300°C, 350°C und 400°C durchgeführt, wobei stets eine Glucosekonzentration von 5 Mass.-% und eine Wasserstoffkonzentration von 100 Mol H₂ pro Mol eingesetzter Glucose in dem Reaktor eingestellt wird. In der nachfolgenden Tabelle 9 sind die eingestellten Parameter aufgelistet.

**Tabelle 9:**

| Experiment | E13 | E14 | E15 | E16 |
|---|---|---|---|---|
| Glucosekonzentration im Reaktor [Mass.-% Glucose in Gesamtmassenstrom Reaktor] | 5 | 5 | 5 | 5 |
| eingesetzte Wasserstoffkonzentration [Mol H₂ pro Mol eingesetzter Glucose] | 100 | 100 | 100 | 100 |
| Verweilzeit im Reaktor [Sekunden, s] | 5 | 5 | 5 | 5 |
| Temperatur im Reaktor [°C] | 250 | 300 | 350 | 400 |
| Druck im Reaktor [bar] | 250 | 250 | 250 | 250 |

Die Experimente E13 bis E16 werden entsprechend Beispiel 1 bis 4 unter Berücksichtigung der vorgenannten Verfahrensparameter durchgeführt.

In der nachfolgenden Tabelle 10 sind die im Rahmen der Experimente erhaltenen Ergebnisse anhand des Umsatzes der als Edukt eingesetzten Glucose sowie anhand der Ausbeuten der als Produkte erhaltenen Zuckeralkohole Sorbit, Mannit und weiterer Polyalkohole zusammengestellt.

**Tabelle 10:**

| Experiment | E13 | E14 | E15 | E16 |
|---|---|---|---|---|
| Wiederfindungsrate an Kohlenstoff (TOC_{Produkte}¹⁾/TOC_{Edukte}¹⁾ in [%]) | 83 | 80 | 54 | 20 |
| Umsatz an Glucose [%] | 84 | 80 | 54 | 20 |
| Ausbeute²⁾ an Sorbit [%] | 41,8 | 11,0 | 1,0 | 0,1 |
| Ausbeute²⁾ an Mannit [%] | 6 | 4,2 | 0 | 0 |
| Ausbeute an C₂-Alkoholen [%] | | 17,2 | 4,9 | 2,0 |
| Ausbeute an C₃-/C₄-Alkoholen [%] | 13 | 29,5 | 13 | 6,5 |

Anmerkung: Als C₂-Alkohol wurde Ethandiol mit den in Tabelle 10 genannten Ausbeuten bestimmt; als C₃-/C₄-Alkohole wurden die in Tabelle 10 genannten Summen der Ausbeuten an Propantriol (Glycerin), 1,3-Propandiol, 2,3-Butandiol und 1,4-Butandiol bestimmt.

Aus den Experimenten E13 bis E16 ist ersichtlich, daß eine Erhöhung der Reaktionstemperatur auf Werte oberhalb 300°C nicht zu einer Erhöhung der Ausbeute an den kurzkettigen Polyolen führt, sondern daß deren Ausbeute vielmehr - bei einem ebenfalls abnehmenden Umsatz der eingesetzten Glucose - abnimmt. Bei 250°C (E13) wird trotz der sehr kurzen Verweilzeit von 5 s noch eine Ausbeute an Sorbit von etwa 42% und an Mannit von 6% erzielt, während etwa 13% Polyole aus der Gruppe Propantriol (Glycerin), 1,3-Propandiol, 2,3-Butandiol und 1,4-Butandiol erhalten werden. Bei 300°C (E14) ergibt sich eine Ausbeute von Sorbit bzw. Mannit von nur noch 11% bzw. etwa 4%, während die Ausbeute der genannten Polyole auf etwa 30% zunimmt. Zugleich wird ein Maximum der Ausbeute an Ethandiol von etwa 17% festgestellt. Bei höheren Temperaturen von 350°C bzw. 400°C (E15 und E16) nehmen die Ausbeuten aller gewünschten Produkte drastisch ab.

### Beispiel 6

Kontinuierliche Herstellung der Zuckeralkohole Sorbit und Mannit und gegebenenfalls weiterer Polyalkohole durch Hydrierung von Fructose in wäßriger Lösung mit gasförmigem Wasserstoff in Gegenwart eines Ruthenium/Rutheniumoxid-Mischkatalysators auf Aluminiumoxid in Form eines in den Reaktor eingebrachten Festbettes:

In dem Rohrreaktor aus Edelstahl mit einer Reaktorlänge von zwei mal 163 cm (parallel geschaltet) mit 9 mm Innendurchmesser gemäß den Beispielen 1 bis 5 wird das Monosaccharid Fructose als Edukt in wäßriger Lösung mit gasförmigem Wasserstoff kontinuierlich zur Umsetzung gebracht. In dem Rohrreaktor befindet sich ein Festbett eines Ruthenium/Rutheniumoxid-Mischkatalysators auf Partikeln von Aluminiumoxid als Träger. Das Experiment wird drei mal jeweils bei 150°C durchgeführt, wobei jeweils zwei mal eine Fructosekonzentration von 20 Mass.-% (Experimente E17 und E18) und einmal eine Fructosekonzentration von 1 Mass.-% (Experiment E19; jeweils bezogen auf den Gesamtmassenstrom) in dem Reaktor eingestellt wird. In der nachfolgenden Tabelle 11 sind die eingestellten Parameter aufgelistet.

**Tabelle 11:**

| Experiment | E17 | E18 | E19 |
|---|---|---|---|
| Fructosekonzentration im Reaktor [Mass.-% Glucose in Gesamtmassenstrom Reaktor] | 20 | 20 | 1 |
| eingesetzte Wasserstoffkonzentration [Mol H₂ pro Mol eingesetzter Fructose] | 25 | 25 | 100 |
| Verweilzeit im Reaktor [Sekunden, s] | 300 | 300 | 300 |
| Temperatur im Reaktor [°C] | 150 | 150 | 150 |
| Druck im Reaktor [bar] | 250 | 250 | 250 |

Zur Durchführung der Experimente E17 bis E19 wird der Reaktor mit dem in diesem in Form des Festbettes enthaltenen hydrierenden Katalysator zunächst angefahren, indem der Reaktor bei dem jeweiligen Reaktionsdruck von 250 bar und der jeweiligen Reaktionstemperatur von 150°C mit einem Strom reinen Wassers beaufschlagt wird. Ist ein konstanter Wasserstrom bei den genannten Druck- und Temperaturbedingungen erreicht worden, wird der Reaktor ferner mit einem Wasserstoffstrom beaufschlagt, um den Hydrierkatalysator zu reduzieren und somit zu aktivieren. Hat sich ein konstanter Wasserstrom und ein konstanter Wasserstoffstrom mit dem jeweiligen, in Tabelle 11 wiedergegebenen Wert eingestellt, wird die Fructoselösung zugesetzt, wobei jeweils der in Tabelle 5 genannte Massenstrom an Fructose eingestellt wird. Dabei wird die Fructosekonzentration im Reaktor wiederum mittels Dosierpumpen und so eingestellt, daß sich ein Wert von 20 (Experimente E17 und E18) bzw. 1 Mass.-% (Experiment E19) bezogen auf den dem Reaktor zugesetzten Gesamtmassenstrom ergibt. Die Verweilzeit von 300 s bzw. 5 min kann ebenfalls - unter Berücksichtigung der Dichte des Mediums bei den im Reaktor herrschenden Bedingungen und dem jeweiligen Volumenstrom - durch entsprechende Steuerung der zugeordneten Dosierpumpe eingestellt werden.

In der nachfolgenden Tabelle 12 sind die im Rahmen der Experimente erhaltenen Ergebnisse anhand des Umsatzes der als Edukt eingesetzten Fructose sowie anhand der Ausbeuten der als Produkte erhaltenen Zuckeralkohole Sorbit, Mannit und weiterer Polyalkohole zusammengestellt.

**Tabelle 12:**

| Experiment | E17 | E18 | E19 |
|---|---|---|---|
| Wiederfindungsrate an Kohlenstoff (TOC_{Produkte}¹⁾/TOC_{Edukte}¹⁾ in [%]) | 98 | 100 | 86,8 |
| Umsatz an Fructose [%] | 100 | 100 | 100 |
| Ausbeute²⁾ an Sorbit [%] | 48,3 | 44,8 | 34,9 |
| Ausbeute²⁾ an Mannit [%] | 61,6 | 59,3 | 36,9 |
| Ausbeute an C₆-Alkoholen [%] | <1 | <1 | < 1 |
| Ausbeute an C₃-/C₄ Alkoholen [%] | <1 | <1 | < 1 |

Die Experimente E17 bis E19 zeigen, daß bei einer Verweilzeit von 300 s, einem Druck von 250 bar und einer Temperatur von 150°C ein vollständiger Fructoseumsatz in Höhe von 100% bei der kontinuierlichen katalytischen Hydrierung der Fructose mit Wasserstoff an Ruthenium/Rutheniumoxid auf Aluminiumoxid erzielt werden kann. Dabei ergeben sich etwa konstante Ausbeuten bzw. Selektivitäten der Zuckeralkohole Sorbit und Mannit im Verhältnis von etwa 2:3, welches auch im Falle einer Erhöhung der Temperatur - im Gegensatz zu einem Einsatz von Glucose als Edukt (vgl. Beispiele 1 ff) - im wesentlichen konstant bleibt, während der Anteil an weiteren Reaktionsprodukten, wie C₃-, C₄- und C₆-Alkoholen, dann ansteigt.

Im Falle einer eingestellten Fructosekonzentration im Reaktor von 1 Mass.-% (Experiment E19) ergeben sich geringfügig schlechtere Ausbeuten an Mannit und Sorbit als bei einer Fructosekonzentration im Reaktor von 20 Mass.-% (Experimente E17 und E18). Ein idealer Bereich kann zwischen etwa 5 Mass.-% und etwa 40 Mass.-% Fructose bezogen auf den Gesamtmassenstrom im Reaktor festgestellt werden.

### Beispiel 7

Kontinuierliche Herstellung der Zuckeralkohole Sorbit und Mannit und gegebenenfalls weiterer Polyalkohole durch Hydrierung von Saccharose (Disaccharid mit einer Glucose- und einer Fructoseeinheit) in wäßriger Lösung mit gasförmigem Wasserstoff in Gegenwart eines Ruthenium/Rutheniumoxid-Mischkatalysators auf Aluminiumoxid in Form eines in den Reaktor eingebrachten Festbettes:

In dem Rohrreaktor aus Edelstahl mit einer Reaktorlänge von zwei mal 163 cm (parallel geschaltet) mit 9 mm Innendurchmesser gemäß den Beispielen 1 bis 6 wird das Disaccharid Saccharose als Edukt in wäßriger Lösung mit gasförmigem Wasserstoff kontinuierlich zur Umsetzung gebracht. In dem Rohrreaktor befindet sich ein Festbett eines Ruthenium/Rutheniumoxid-Mischkatalysators auf Partikeln von Aluminiumoxid als Träger. Das Experiment wird insgesamt drei mal, d.h. einmal bei 150°C (Experiment E20), einmal bei 200°C (E21) und einmal bei 250°C (E22), durchgeführt, wobei die eingestellten Versuchsparameter in der nachfolgenden Tabelle 13 zusammengefaßt sind.

**Tabelle 13:**

| Experiment | E20 | E21 | E22 |
|---|---|---|---|
| Saccharosekonzentration im Reaktor [Mass.-% Glucose in Gesamtmassenstrom Reaktor] | 1 | 5 | 5 |
| eingesetzte Wasserstoffkonzentration [Mol H₂ pro Mol eingesetzter Saccharose] | 300 | 300 | 300 |
| Verweilzeit im Reaktor [Sekunden, s] | 300 | 300 | 300 |
| Temperatur im Reaktor [°C] | 150 | 200 | 250 |
| Druck im Reaktor [bar] | 250 | 250 | 250 |

Zur Durchführung der Experimente E20 bis E22 wird der Reaktor mit dem in diesem in Form des Festbettes enthaltenen hydrierenden Katalysator zunächst angefahren, indem der Reaktor bei dem jeweiligen Reaktionsdruck von 250 bar und der jeweiligen Reaktionstemperatur von 150°C, 200°C bzw. 250°C mit einem Strom reinen Wassers beaufschlagt wird. Ist ein konstanter Wasserstrom bei den genannten Druck- und Temperaturbedingungen erreicht worden, wird der Reaktor ferner mit einem Wasserstoffstrom beaufschlagt, um den Hydrierkatalysator zu reduzieren und somit zu aktivieren. Hat sich ein konstanter Wasserstrom und ein konstanter Wasserstoffstrom mit den jeweiligen, in Tabelle 13 wiedergegebenen Werten eingestellt, wird die Saccharoselösung zugesetzt, wobei jeweils der in Tabelle 13 genannte Massenstrom an Saccharose eingestellt wird. Dabei wird die Saccharosekonzentration im Reaktor wiederum mittels Dosierpumpen und so eingestellt, daß sich ein Wert von 5 (Experimente E21 und E22) bzw. 1 Mass.-% (Experiment E20) bezogen auf den dem Reaktor zugesetzten Gesamtmassenstrom ergibt. Die Verweilzeit von 300 s bzw. 5 min kann ebenfalls - unter Berück- sichtigung der Dichte des Mediums bei den im Reaktor herrschenden Bedingungen und dem jeweiligen Volumenstrom - durch entsprechende Steuerung der zugeordneten Dosierpumpe eingestellt werden.

In der nachfolgenden Tabelle 14 sind die im Rahmen der Experimente erhaltenen Ergebnisse anhand des Umsatzes der als Edukt eingesetzten Saccharose sowie anhand der Ausbeuten der als Produkte erhaltenen Zuckeralkohole Sorbit und Mannit zusammengestellt .

**Tabelle 14:**

| Experiment | E20 | E21 | E22 |
|---|---|---|---|
| Wiederfindungsrate an Kohlenstoff (TOC_{Produkte}¹⁾TOC_{Edukte}¹⁾ in [%]) | 88 | 93 | 30 |
| Umsatz an Saccharose [%] | 59 | 98 | 100 |
| Ausbeute²⁾ an Sorbit [%] | 49 | 64, 5 | 5,1 |
| Ausbeute²⁾ an Mannit [%] | 18 | 35,5 | 6,2 |

Die Experimente E20 bis E22 zeigen, daß bei einer Verweilzeit von 300 s, einem Druck von 250 bar und einer Temperatur von 200°C und 250°C (E21 und E22) ein praktisch vollständiger Saccharoseumsatz im Bereich von 100% bei der kontinuierlichen katalytischen Hydrierung der Saccharose mit Wasserstoff an Ruthenium/Rutheniumoxid auf Aluminiumoxid erzielt werden kann. Bei einer Temperatur von 150°C werden im Falle des eingesetzten Disaccharides Saccharose gegenüber einer Temperatur von 200°C schlechtere Ausbeuten an Sorbit und Mannit bei einem Umsatz von lediglich etwa 60% erhalten, was vermutlich darauf zurückzuführen ist, daß die Saccharose bei dieser Temperatur nicht vollständig in Glucose und Fructose aufgespalten wird. Dies gilt sowohl für eine eingestellte Saccharosekonzentration im Reaktor von 1 Mass.-%, wie es gemäß Experiment E20 der Fall ist, als auch für eine den Experimenten E21 und E22 entsprechende Saccharosekonzentration von 5 Mass.-% im Reaktor (nicht im einzelnen wiedergegeben; jeweils bezogen auf den Gesamtmassenstrom). Ein optimaler Parameterbereich der Temperatur beträgt im Falle eines gewünschten Produktes in Form von Sorbit und Mannit zwischen etwa 175°C und etwa 225°C, insbesondere in der Größenordnung von etwa 200°C (E21).

In dem genannten Temperaturintervall ergeben sich Ausbeuten der Zuckeralkohole Sorbit und Mannit - hier im Verhältnis zwischen etwa 3:1 und 2:1 -, wobei das Produktverhältnis mit steigender Temperatur in Richtung zu Mannit hin verschoben werden kann. Im Falle einer Erhöhung der Temperatur auf 250°C (E22) erhöht sich der Anteil an hier nicht näher wiedergegebenen weiteren Reaktionsprodukten, wie C₃-, C₄- und C₆-Alkoholen, vornehmlich auf der Gruppe 1,3-Propandiol und Propantriol (Glycerin) sowie 2,3-Butandiol und 1,4-Butandiol, erheblich; die Ausbeuten an Sorbit bzw. Mannit betragen nur noch ca. 5,1% bzw. 6,2%.

### Fazit

Das erfindungsgemäße Verfahren bietet somit einerseits eine effektive Herstellung der Zuckeralkohole Sorbit und Mannit mittels eines über sehr lange Zeiträume aktiven und leicht handhabbaren Katalysators bei sehr kurzen Reaktionszeiten und einem vollständigen Umsatz der Edukte unter Gewährleistung einer einfachen Einstellbarkeit des gewünschten Produktspektrums Mannit und Sorbit, andererseits eine ebenfalls effektive Herstellung weiterer Polyole, insbesondere aus der Gruppe Propantriol (Glycerin), 1,3-Propandiol, 2,3-Butandiol und 1,4-Butandiol sowie Ethandiol, aus nachwachsenden Rohstoffen, wobei hieraus hergestellte Polyurethane gegenüber Polyurethanen, welche aus entsprechenden, aus Erdöl/Erdgas gewonnenen Polyolen erzeugt worden sind, verbesserte Werkstoffeigenschaften aufweisen können.

## Patentansprüche

1. Verfahren zur Herstellung von Polyalkoholen in Form von Zuckeralkoholen aus der Gruppe Sorbit und/oder Mannit und gegebenenfalls weiteren C₆- und/oder C₄- und/oder C₃-Polyolen und/oder C₂-Polyolen, wobei wenigstens ein wenigstens eine Glucose- und oder wenigstens eine Fructoseeinheit enthaltendes Mono-, Di-, Oligo- und/oder polysaccharid in Gegenwart eines hydrierenden Katalysators auf der Basis von Ruthenium (Ru) und/oder Rutheniumoxid in wäßriger Phase bei erhöhter Temperatur und erhöhtem Druck mit Wasserstoff unter Erhalt der genannten Polyalkohole kontinuierlich zur Umsetzung gebracht wird, wobei eine Temperatur zwischen 120°C und 280°C, ein Druck von wenigstens 150 bar und eine Verweilzeit der Reaktanden bei der katalytischen Hydrierung von höchstens 400 s eingestellt wird.

2. Verfahren nach Anspruch 1, **dadurch gekennzeichnet, daß** eine Verweilzeit von 5 s bis 360 s eingestellt wird.

3. Verfahren nach Anspruch 1 oder 2, **dadurch gekennzeichnet, daß** ein Druck von wenigstens 200 bar, insbesondere von wenigstens 220 bar, eingestellt wird.

4. Verfahren nach einem der Ansprüche 1 bis 3, **dadurch gekennzeichnet, daß** die Produktanteile der Zuckeralkohole Sorbit und Mannit und gegebenenfalls der C₆- und/oder C₄- und/oder C₃-Polyole und/oder C₂-Polyole durch Variation wenigstens eines der Parameter Temperatur, insbesondere in einem Bereich gemäß Anspruch 5, Druck, Verweilzeit und/oder Wasserstoff- bzw. Eduktkonzentration eingestellt werden.

5. Verfahren nach einem der Ansprüche 1 bis 4, **dadurch gekennzeichnet, daß** als Edukt Glucose und/oder Fructose und/oder ein sowohl wenigstens eine Glucose- als auch wenigstens eine Fructoseeinheit enthaltendes Di-, Oligo- oder Polysaccharid, insbesondere Saccharose und/oder Raffinose, eingesetzt wird.

6. Verfahren nach einem der Ansprüche 1 bis 5, **dadurch gekennzeichnet, daß** eine Eduktkonzentration zwischen 1 und 50 Mass.-%, insbesondere zwischen 2 und 40 Mass. -%, Edukte bezogen auf den Gesamtstrom eingestellt wird.

7. Verfahren nach einem der Ansprüche 1 bis 6, **dadurch gekennzeichnet, daß** als Katalysator ein Ruthenium/Rutheniumoxid-Mischkatalysator eingesetzt wird.

8. Verfahren nach einem der Ansprüche 1 bis 7, **dadurch gekennzeichnet, daß** ein an wenigstens einem Träger immobilisierter Katalysator eingesetzt wird.

9. Verfahren nach Anspruch 8, **dadurch gekennzeichnet, daß** als Katalysatorträger Aluminiumoxid (Al₂O₃) eingesetzt wird.

## Claims

1. Method for the production of polyalcohols in the form of sugar alcohols from the group sorbite and/or mannitol and if necessary further C₆ and/or C₄ and/or C₃ polyols and/or C₂ polyols, at least one mono-, di-, oligo- and/or polysaccharide, which contains at least one glucose and or at least one fructose unit, being brought continuously to conversion in the presence of a hydrogenating catalyst based on ruthenium (Ru) and/or ruthenium oxide in aqueous phase at increased temperature and increased pressure with hydrogen with production of the mentioned polyalcohols, a temperature between 120°C and 280°C, a pressure of at least 150 bar and a dwell time of the reactands during the catalytic hydrogenation of at most 400 s being set.

2. Method according to claim 1, **characterised in that** a dwell time of 5 s to 360 s is set.

3. Method according to claim 1 or 2, **characterised in that** a pressure of at least 200 bar, in particular of at least 220 bar, is set.

4. Method according to one of the claims 1 to 3, **characterised in that** the product proportions of the sugar alcohols sorbite and mannitol and if necessary of the C₆ and/or C₄ and/or C₃ polyols and/or C₂ polyols arc adjusted by variation of at least one of the parameters, temperature, in particular in a range according to claim 5, pressure, dwell time and/or hydrogen- or educt concentration.

5. Method according to one of the claims 1 to 4, **characterised in that** glucose and/or fructose and/or a di-, oligo- or polysaccharide, which contains both at least one glucose and at least one fructose unit, in particular saccharose and/or raffinose, is used as educt.

6. Method according to one of the claims 1 to 5, **characterised in that** an educt concentration between 1 and 50% by mass, in particular between 2 and 40% by mass, of educts relative to the total flow is set.

7. Method according to one of the claims 1 to 6, **characterised in that** a ruthenium/ruthenium oxide mixed catalyst is used as catalyst.

8. Method according to one of the claims 1 to 7, **characterised in that** a catalyst which is immobilised on at least one carrier is used.

9. Method according to claim 8, **characterised in that** aluminium oxide (Al₂O₃) is used as catalyst carrier.

## Revendications

1. Procédé d'obtention de polyalcools sous forme d'alcools de sucre du groupe du sorbitol et/ou manitol et le cas échéant d'autres polyols en C₆ et/ou C₄ et/ou C₃ et/ou polyols en C₂, selon lequel on fait réagir en continu au moins un mono⁻, di⁻, oligo⁻ et/ou polysaccharide renfermant au moins une unité glucose et/ou au moins une unité fructose avec de l'hydrogène en présence d'un catalyseur d'hydrogénation à base de ruthénium (Ru) et/ou d'oxyde de ruthénium, en phase aqueuse, à température élevée et sous pression élevée pour obtenir les polyalcools susmentionnés, et on règle une température comprise entre 120°C et 280°C, une pression d'au moins 150 bars et un temps de séjour des produits réactionnels d'au maximum 400 s lors de l'hydrogénation catalytique.

2. Procédé selon la revendication 1,
**caractérisé en ce que**
l'on règle un temps de séjour de 5 s à 360 s.

3. Procédé selon la revendication 1 ou la revendication 2,
**caractérisé en ce que**
l'on règle une pression d'au moins 200 bars, en particulier d'au moins 220 bars.

4. Procédé selon l'une des revendications 1 à 3,
**caractérisé en ce que**
l'on règle les fractions des alcools de sucre, sorbitol et manitol et le cas échéant des polyols en C₆ et/ou C₄ et/ou C₃ et/ou des polyols en C₂ en faisant varier au moins l'un des paramètres de température -en particulier dans une plage selon la revendication 5- de pression, de temps de séjour et/ou de concentration en hydrogène ou en éduit.

5. Procédé selon l'une des revendications 1 à 4,
**caractérisé en ce qu'**
en tant qu'éduit, on met en oeuvre du glucose et/ou du fructose et/ou un di-, oligo- ou polysaccharide renfermant au moins une unité glucose ainsi qu'au moins une unité fructose, en particulier du saccharose et/ou du raffinose.

6. Procédé selon l'une des revendications 1 à 5,
**caractérisé en ce que**
l'on règle une concentration en éduit comprise entre 1 % et 50 % en masse, en particulier entre 2 % et 40 % en masse d'éduits par rapport au flux total.

7. Procédé selon l'une des revendications 1 à 6,
**caractérisé en ce qu'**
en tant que catalyseur, on met en oeuvre un catalyseur mixte ruthénium/oxyde de ruthénium.

8. Procédé selon l'une des revendications 1 à 7,
**caractérisé en ce qu'**
on met en oeuvre un catalyseur immobilisé sur au moins un support.

9. Procédé selon la revendication 8,
**caractérisé en ce qu'**
en tant que support de catalyseur, on met en oeuvre de l'oxyde d'aluminium (Al₂O₃).
